# EUROPEAN PATENT SPECIFICATION

(11) **EP 0 832 207 B1**
(45) Date of publication and mention of the grant of the patent: **13.09.2000**
(21) Application number: 96916019.1
(22) Date of filing: 31.05.1996
(51) Int. Cl.: C12N 15/10, C12N 15/86, C12Q 1/68, G01N 33/68

(54) **A METHOD FOR IDENTIFICATION OF BIOLOGICALLY ACTIVE PEPTIDES AND NUCLEIC ACIDS**
VERFAHREN ZUR IDENTIFIKATION VON BIOLOGISCH AKTIVEN PEPTIDEN UND NUKLEINSÄUREN
PROCEDE D'IDENTIFICATION DE PEPTIDES ET D'ACIDES NUCLEIQUES BIOLOGIQUEMENT ACTIFS

(30) Priority: 02.06.1995 DK 62995
(43) Date of publication of application: 01.04.1998
(60) Divisional application: 00201278.9
(73) Proprietor: M&E BIOTECH A/S, 2970 Horsholm (DK)
(72) Inventor: JENSEN, Martin, Roland, DK-2840 Holte (DK); PEDERSEN, Finn, Skou, DK-8210 Aarhus V (DK); MOURITSEN, Soeren, DK-3460 Birkeroed (DK); HINDERSSON, Peter, DK-1777 Copenhagen V (DK); DUCH, Mogens, DK-8240 Risskov (DK); SOERENSEN, Michael, Schandorf, DK-8000 Aarhus (DK); DALUM, Iben, DK-2970 Hoersholm (DK); LUND, Anders, Henrik, DK-8000 Aarhus C (DK)
(74) Representative: Plougmann, Vingtoft & Partners A/S
(86) International application number: DK9600231
(87) International publication number: WO9638553

(56) References cited:
- WO-A-95/04824
- BIO/TECHNOLOGY, Volume 12, October 1994, MARTA DUENAS et al., "Clonal Selection and Amplification of Phage Displayed Antibodies by Linking Antigen Recognition and Phage Replication", page 999.
- DIALOG INFORMATION SERVICES, File 154, MEDLINE, Dialog Accession No. 08151796, Medline Accession No. 92289796, SCHUMACHER T.N. et al., "Synthetic Peptide Libraries in the Determination of T Cell Epitopes and Peptide Binding Specificity of Class I Molecules"; & EUR. J. IMMUNOL., (GERMANY), Jun. 1992, 22(6), p. 1405-12.

## Description

This invention concerns a novel method for identification of new peptides and post-translationally modified peptides as well as nucleic acids with biological activity.

### BACKGROUND OF THE INVENTION

During the last five years the technology for expressing, testing and identifying millions of different random peptide sequences has evolved dramatically. Such peptide libraries can be used for identification of new biologically active peptides, and therefore the technology has added an exciting and very promising new epoch to the field of drug development.

The known peptide library techniques can at present be divided into two fundamentally different groups: The random synthetic peptide libraries, in which the random peptides are produced chemically, and the random biosynthetic peptide libraries, in which the random peptides are encoded by partly or totally random DNA sequences and subsequently synthesized by ribosomes.

### The synthetic peptide libraries.

Synthetic peptide libraries containing millions of peptides can be produced by combinatorial peptide chemistry and may either be synthesized in soluble form (R.A. Houghten et al. Nature, 354, 84-86, 1991) or remain immobilized on the peptide resin beads (A. Furka et al., Int. J. Peptide Protein Res. 37, 487-493, 1991; K. S. Lam et al., Nature, 354, 82-84, 1991). Using either of these approaches different receptor ligands have been isolated.

The advantage of the soluble peptide libraries compared to solid phase immobilized peptide libraries is that soluble peptides may bind more sterically unhindered to the receptors in question. In the synthetic peptide library technique proposed by Furka et al. 1991 and Lam et al. 1991, respectively, the most important improvement, on the other hand, was the approach of having only one type of peptide sequence on each bead ("One bead - one peptide"). This enables direct selection and eventually sequencing of the putative active peptide ligand on a single bead using e.g. Edman degradation. Using this technology active peptides including peptides consisting of D-amino acids or other unnatural amino acids can be identified (B. Gissel et al. J. Peptide Science. In Press, 1995).

### The biosynthetic peptide libraries.

Bacteriophage expression vectors have been constructed that can display peptides on the phage surface (S.E. Cwirla et al, Proc. Natl. Acad. Sci. USA, 87, 6378-6382, 1990). Each peptide is encoded by a randomly mutated region of the phage genome, so sequencing of the relevant DNA region from the bacteriophage found to bind a receptor will reveal the amino acid sequence of the peptide ligand. A phage containing a peptide ligand is detected by repeated panning procedures which enrich the phage population for a strong receptor binding phage (J.K. Scott, TIBS, 17, 241-245, 1992).

Bacteria have also been used for expression of similar peptide libraries. The peptides can be fused to an exported protein, such as an antibody, which can be immobilized on a solid support. By screening the solid support with an appropriate soluble receptor the bacterial clone producing the putative peptide ligand can be identified (M.G. Cull et al, Proc. Natl. Acad. Sci. USA, 89, 1865-1869, 1992).

Another described method for preparing large pools of different possibly active compounds is by the use of libraries consisting of randomized ribonucleotides or deoxyribonucleotides, the so-called aptamer libraries. The aptamers are generated in E. coli from a plasmid vector containing randomized DNA. From these libraries structures with biological activity have been identified (L.C. Bock et al, Nature, 355, 564-566, 1992).

### PURPOSE OF THE INVENTION

In order to use the prior art methods for identifying biologically active peptides and nucleic acids or their respective cellular target proteins, it is necessary to possess a detailed knowledge about the molecular mechanisms involved in a certain biological process. If these mechanisms are known, it may subsequently be possible to develop antagonists or agonists of targets (receptors, enzymes, etc.) involved using said methods. The problem to be solved by the present invention is i.a. to overcome the need for said detailed knowledge.

### SUMMARY OF THE INVENTION

According to the present invention the peptide sequences, or the ribonucleic acids are identified from biosynthetically expressed eukaryotic libraries containing millions of partly or totally random peptides and ribonucleic acids. In connection with this invention the term "peptide" shall be understood to comprise also a peptide sequence introduced into or fused to a larger protein (e.g. an antibody). The peptides and ribonucleic acids are synthesized by the cells from random DNA sequences which have been effectively transduced into the cells. Some of the peptides or ribonucleic acids in the library will affect important biological functions in the cells which express them. Cells which change phenotype due to the presence of such substances can be isolated, and their chemical structure can subsequently be clarified by sequencing of the DNA which encodes them. Such peptides could possibly be used therapeutically or as lead compounds for future drug development or they can be used for identification of new target proteins which are causing the change in the biological function of the cell. Such target proteins can eventually be used in the development of drugs by e.g. conventional medicinal chemistry or synthetic peptide libraries.

Accordingly, the method of the invention comprises a method for identification of biologically active ribonucleic acids or peptides or for isolation of cellular ligands to the biologically active ribonucleic acids or peptides, which comprises the steps of
(a) production of a pool of appropriate vectors each containing a DNA sequence to be examined,
(b) efficient transduction of said vectors into a number of identical eukaryotic cells in such a way that each cell expresses either a single ribonucleic acid and possibly peptide encoded by the DNA sequence to be examined or a limited number of different ribonucleic acids and peptides encoded by DNA sequences to be examined,
(c) screening of said transduced cells to see whether some of them have changed a certain phenotypic trait, and
(d) selection and cloning of said changed cells,
characterized in that
the pool of appropriate vectors in step (a) contains totally or partly random DNA sequences selected from the group consisting of:
i) synthetic random DNA sequences obtainable by conventional random oligonucleotide synthesis;
ii) synthetic random DNA sequences wherein stop codons are absent and which encode random amino acid sequences with an even distribution of amino acids;
iii) synthetic DNA sequences defined in(i) or (ii) including codons which enable specific post-translational modifications of all expressed peptides or which encode anchor residues;
iv) synthetic DNA sequences defined in (i), (ii), or (iii) fused to or having inserted therein coding sequences for purification tags which facilitate the purification and identification of expressed peptides; and
v) synthetic DNA sequences defined in (i), (ii), (iii), or (iv) inserted into or fused to the coding sequence of a protein;
and wherein
(e) the vector DNA in the phenotypically changed cells is isolated and sequenced, and the sequences of the biologically active ribonucleic acids or peptides are deduced from the sequenced vector DNA;
and/or
(f) the biologically active ribonucleic acids or peptides expressed in the phenotypically changed cells are used directly for isolation of a ligand molecule to said ribonucleic acids or peptides.

### BRIEF DESCRIPTION OF THE DRAWING

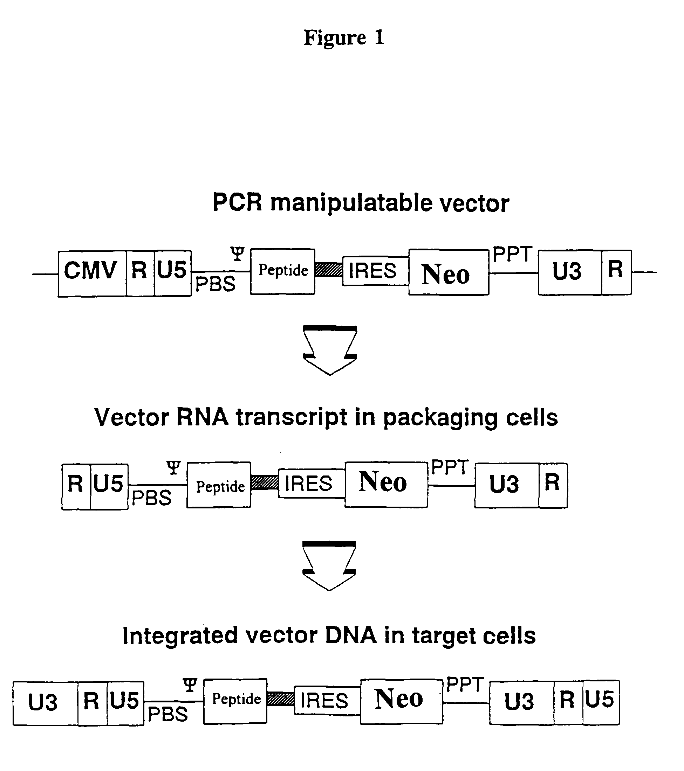

Figure 1 is a schematic drawing of a standard retroviral peptide expression vector. The plasmid form of the vector (top) carries a cytomegalovirus (CMV) promoter directing expression of a retroviral RNA (middle) with a backbone (R, U5, PBS, Ψ, PPT, U3, and R) from Akv murine leukaemia virus and a peptide translation cassette followed by an internal ribosomal entry site (IRES) from EMC virus directing translation of a Neomycin resistance gene (Neo). The vector provirus in the target cell (below) contains a regenerated retroviral long terminal repeat - LTR (U3, R, and U5). The CMV promoter sequence provides a unique tag for efficient initial PCR mutagenesis and amplification. The size of the vector without an inserted peptide expression cassette is 4.0 kb.

### DESCRIPTION OF THE PREFERRED EMBODIMENTS

A built-in requirement of all the presently known peptide library techniques is the necessity of a detailed knowledge about the mechanisms by which a given receptor or enzyme regulates a certain phenotypic trait of the cell. This receptor or enzyme furthermore has to be available in a relatively pure form before a ligand can be selected in either of the two types of peptide libraries. When potential peptide ligands eventually have been identified, functional assays have to be performed to determine whether the ligands exert antagonistic or agonistic effects on the desired cellular phenotypic trait.

The method according to the present invention overcomes this major problem. By the present method it is thus not necessary to know the chain of mechanisms, receptors, signalling pathways, enzymes etc. which generate the phenomenon inside or on the surface of the cell since it is the resulting biological effect or phenotypic trait which is screened for. This is achieved by the following steps: (a) production of a pool of appropriate vectors each containing totally or partly random DNA sequences, (b) efficient transduction of said vectors into a number of identical eukaryotic, e.g. mammalian, cells in such a way that only a single ribonucleic acid and peptide species is expressed ("one cell - one ribonucleic acid or peptide") or optionally a limited number of different random ribonucleic acids and peptides are expressed by each cell, (c) screening of said transduced cells to see whether some of these have changed a certain phenotypic trait, (d) selection and cloning of said changed cells, (e) isolation and sequencing of the vector DNA in said phenotypically changed cells, and (f) deducing the RNA and peptide sequences from the DNA sequence. Either the RNA or the peptide encoded by the isolated DNA sequences may be the cause of said phenotypic changes of the cell and may therefore possess biological activity.

The introduced peptides are expressed e.g. in the cytoplasm of biologically interesting cells, which before that were totally identical, using e.g. the retroviral vector systems described in Example 1. They are expressed from a pool of vectors containing random DNA sequences which has been constructed e.g. as described in Example 1. Using an appropriate ratio between infective retrovirus and non-infected cells only a single DNA copy derived from the pool of retrovirus vectors is introduced into each cell. The major advantage by this "one cell - one ribonucleic acid or peptide" concept is that cells which have changed phenotypically upon the introduction of peptides can be isolated by cloning and selection methods, and that the active peptide causing the phenotypic change can subsequently be identified. This is accomplished by isolating the DNA fragment encoding the peptide, e.g. by Polymerase Chain Reaction (PCR) technology, and subsequently identifying the DNA sequence.

During the initial screening procedure a larger number of retrovirus vectors can be introduced into each cell which enables the individual cell to express a number of different ribonucleic acids or peptides. When a phenotypically changed cell clone subsequently has been isolated all retroviral DNA in that particular clone can be isolated by PCR, and the PCR product can be used for retransfection of the packaging cells ordinarily used for virus production. The retroviral vectors isolated from these packaging cells can subsequently be used for transduction of new biologically interesting cells using the "one cell - one ribonucleic acid or peptide" concept. Finally, after a second cloning procedure the active substance can be identified as described above. Further, the biologically active ribonucleic acid, peptide or protein isolated by this method can be utilized either as a lead compound for drug development or as an affinity ligand with the purpose of isolating and identifying the protein target responsible for the biological activity. Such target proteins are very useful tools in drug development.

The use of small vectors (below 3 kb of DNA) has the major advantage of allowing simple PCR-mutagenesis and amplification without ligation and cloning steps. Another important advantage of using small vectors is that the vectors in a pool of target cells can be amplified directly by PCR and retransfected into packaging cells, hence allowing multiple rounds of selection to remove time-consuming analysis of false positives or contaminating cells. Direct sequence analysis of the derived random plasmid clones is used to assure the randomness of the expression library. The standard vector capable of expressing the random peptide library is shown schematically in Fig. 1.

The cells which are found to have changed phenotypically upon the introduction of the random DNA sequences could alternatively have changed as a consequence of interactions with the ribonucleic acid molecule transcribed from the introduced DNA, in analogy to the described libraries consisting of randomized ribonucleotides or deoxyribonucleotides, the so-called aptamer libraries. Such ribonucleic acid molecules would therefore also possess biological activity. Furthermore, the observed effect could also be due to biological activity of carbohydrate moieties or other post-translational modifications on the expressed peptides in the cell. Using an appropriate purification-tag on the peptides in the library, it would be possible in that case to purify these and analyze the exact chemical structure of the post-translational modifications in question.

Since the efficiency of the non-viral methods commonly used for stable gene transfer into mammalian cells is very low, it would not be possible to establish a peptide expression library in mammalian cells by such methods. In addition non-viral methods generally lead to multiple integrations of DNA in the cell genome in disagreement with the "one cell one ribonucleic acid or peptide" concept. In order to achieve the necessary high efficiency single gene copy transfer a viral vector must be used. Very recently cDNA expression libraries which were constructed using retroviral vectors have been described. From such libraries cytokines and cellular growth factors have been isolated (A.J.M. Murphy et al., Proc. Natl. Acad. Sci. USA, 84, 8277-8281, 1987., B.Y. Wong et al., J. Virol., 68, 5523-31, 1994., J.R. Rayner et al., Mol. Cell. Biol., 14, 880-887, 1994). Expression of well defined peptides in transfected eukaryotic cells has also previously been established, although not using retroviral vectors (M.S. Malnati et al., Nature, 357, 702-704, 1992., E.O. Long et al., J. Immunol., 153, 1487-1494, 1994). A library of random peptides has never been expressed in mammalian cells with the purpose of identifying biologically active peptides or ribonucleic acids.

Immunology is an important biological field where the method according to the invention can be used. T cells only recognize fragments of protein antigens, and only if these are bound to MHC molecules. Two types of MHC molecules - the class I and II molecules - present antigen fragments to T cells. The peptides presented by MHC class I molecules, which are on the surface of essentially all nucleated cells, are 8-9 amino acids long and generally derived from proteins in the cytosol of the cell. These can be self-proteins, viral proteins, peptides introduced into the cytosol by transfection or tumor antigens. It is of considerable interest to be able to identify such peptides or T cell epitopes e.g. with regard to vaccine development or in immunotherapy of cancer. Identification of such fragments is, however, a very difficult, demanding and some times impossible task requiring large amounts of affinity purified MHC molecules derived from the tumor cell in question and iterative combinations of advanced mass spectrometry, HPLC and functional T cell assays. Furthermore, most peptide antigens cannot be identified due to the presence of very low amounts of the individual peptides on the MHC molecules. In Example 2 it is demonstrated that the method according to the present invention can be used for identification of said T cell epitopes.

Cell lines expressing biologically important surface molecules can also be transduced with a random peptide library according to the invention. An example of such molecules could be the B7 co-stimulatory molecule, which is known to be important for activation of T cells, or the selectin family of proteins which are known to be involved in the homing of inflammatory cells to inflamed tissue. Cells which change phenotype (e.g. either up- or down-regulate B7) can be selected and cloned as described in Example 3. After isolation of the transduced DNA by PCR new cells can be transduced with the isolated DNA to confirm the observation. Subsequently, the peptide sequence can be deduced from the DNA sequence.

It has been described by others that specific monoclonal antibodies or F(ab) fragments can be expressed in the cytoplasm of a cell and exert a biological activity there (T.M. Werge et al., Febs Letters, 274, 193-198, 1990).

According to the present invention the wholly or partly random peptide sequence can also be introduced into the variable region of an antibody F(ab) fragment. Therefore, a library of F(ab) fragments containing random peptide sequences can be expressed in a cell clone in a way that each all express a single antibody specificity. Subsequently biologically changed cells are isolated and cloned, and the identified intracellular F(ab) fragment can be used for purification of the target protein involved in the biological proteins. Such target protein can subsequently be used for development of drugs capable of modifying said target proteins.

The invention is illustrated by the following examples:

### EXAMPLE 1

### Construction of an intracellular eukaryotic peptide library

A retrovirus vector capable of expressing random peptide sequences is constructed. If the random DNA sequences used in the vector were produced using conventional random oligonucleotide synthesis a large number of stop codons inevitably would be introduced. Furthermore, due to the degeneracy of the genetic code an uneven distribution of the encoded amino acids would be the result. We avoid this by producing the random DNA sequences by random codon synthesis: An appropriate amount of resin used for solid phase oligonucleotide synthesis (optionally already containing a DNA sequence corresponding to an appropriate vector cloning site) is divided into 20 different portions. On portion no. 1 a conventional solid phase chemical synthesis of three bases corresponding to a codon encoding the amino acid, alanine, is performed. On portion no. 2 a codon encoding cysteine is synthesized and so forth. When each of the 20 portions have been coupled with codons corresponding to each of the natural amino acids, all portions are mixed and divided again into 20 equally sized resin portions. The codon synthesis is then repeated again, and the whole procedure is repeated until the desired randomized DNA sequence has been synthesized - e.g. corresponding to 6-10 random amino acids. This can also be achieved by using blocked and protected trinucleotide phosphoramidites encoding the 20 natural amino acids in a total random oligonucleotide synthesis (J. Sondek et al., Proc. Natl. Acad. Sci. USA, 89, 3581-5, 1992). Finally, other appropriate vector cloning sites can be synthesized on all the oligonucleotides before they eventually are cleaved from the resin.

The pool of random synthetic oligonucleotides can be used to generate a pool of vectors with random sequences in appropriate positions either by restriction cleavage and ligation or, preferentially, to avoid inefficient ligation steps, by PCR-mutagenesis. The procedures follow the principles of site-directed PCR-mediated mutagenesis (S. Perrin et al., Nucl. Acids Res. 18, 7433-38, 1990), but the methodology has been adapted to deliver a complex mixture of products. Briefly, the random oligonucleotides carrying vector sequences flanking the random sequence are used as primers in a PCR reaction together with a unique terminal vector primer. In order to retain complexity large quantities of template as well as of vector and randomized primers are used, and product diversity is further ensured by pooling of multiple independent PCR-reactions. Subsequently, an overlapping PCR fragment containing the remaining vector segment is produced by standard PCR. Finally, this overlapping segment is joined with the PCR fragments containing the random DNA using another unique set of terminal primers.

DNA fragments produced by Taq DNA polymerase enzyme may contain additional nucleotides at the 3'DNA strand. These extra nucleotides will be deleterious for combining two PCR products with overlapping termini into one fragment. By addition of Klenow DNA polymerase enzyme these nucleotides can be removed by the 3' go 5' exonuclease activity of said enzyme, increasing the combining efficiency.

Alternatively the PCR product can be trimmed at the termini by digestion with restriction enzymes whose recognition sequences have been incorporated into the oligonucleotides used as primers for the PCR reaction. By utilization of the temperature cycle method developed by (Lund et al., Nucleic Acids Res., 24, 800-801, 1996) the efficiency of the ligation reaction can be increased and the ligation product used for direct transfection of the packaging cells.

To maintain diversity, the PCR-generated linear vector DNA is used directly for transfection of packaging cells, and virions containing the pool of different vectors are harvested under transient conditions. Small bicistronic single transcript vectors containing a random peptide translation cassette followed by an internal ribosomal entri site (IRES) from EMC virus directing translation of a Neomycin (Neo) resistance gene. The Neo gene functions as a selection marker to allow titre determination and elimination of non-transduced cells, if necessary. Other available relevant vectors employ other selectable genes such as phleomycin and hygromycin B resistance and have the peptide translation cassette after the IRES element. Alternatively, even smaller vectors, carrying only the peptide expression cassette and lacking a selection marker can also be used.

The use of small vectors (below 4 kb of DNA) has the major advantage of allowing simple PCR-mutagenesis and amplification without ligation and cloning steps. Another important advantage of using small vectors is that the vectors in a pool of target cells can be amplified directly by PCR and retransfected into packaging cells, hence allowing multiple rounds of selection to remove time-consuming analysis of false positives or contaminating cells. Direct sequence analysis of the derived random plasmid clones is used to assure the randomness of the expression library. The standard vector capable of expressing the random peptide library is shown schematically in Fig. 1.

To maintain diversity of the vector pool a high fraction of the vector RNA transcripts must be encapsidated into retroviral particles. By transfection of the packaging cells with a DNA construct expressing a tRNA matching the corresponding PBS in the retroviral vector we can increase the production of functional vector containing virus particles 10 fold under transient conditions..

A new packaging cell line will be generated after a single transfection of a construct encoding all retroviral proteins. To diminish the risk of generation of replication competent virus and to obtain maximal expression the vector will have the following simplified outline: promoter-gag-pol transcript-IRES-phleomycin resistance gene-IRES-env-polyadenylation signal. One advantange of said vector is that the phleomycin resistance gene enables selection for high expression and that the sheer size of the vector transcript restricts the encapsidation into retroviral particles thereby diminishing the risk of generation of replication competent virus. The size limit for encapsidation of RNA transcripts is about 10 kb.

In addition to traditional packaging cell line a semi-packaging cell line with a corresponding minivirus-vector will be used. The semi-packaging cell line consists of vectors encoding two mutated gag-pol transcripts complementing each other. The use of two different gag-pol transcripts reduces the risk of generating wild type virus. Each cell in the semi-packaging cell line now contains all retroviral proteins needed for production of retroviral particles except the envelope proteins these proteins are supplied by the minivirus-vector. This vector is a bicistronic vector with following outline LTR-PBS-packaging signal-random peptide-IREs-env-polypurine tract-LTR. This vector will be able to transduce the semi-packaging cells as these do not produce envelope proteins prior to infection with the minivirus-vector.

Thus, infection of the semi-packaging cell will not be restricted by receptor interference.

Restrictions upon the "randomness" of the peptide sequence can be introduced, for the purpose of limiting the number of available sequences in the mammalian cellular library and for introduction of e.g. N-glycosylation sites, or other post-translational modifications of all expressed peptides if so desired. Purification tags - e.g. poly-His or others - can also be included in the expressed peptides for facilitating the purification and identification of the peptide itself. This is necessary for the identification of post-translational modifications, which were not obvious from the peptide sequence.

A population of eukaryotic cells is infected with the retrovirus carrying genetic constructs containing random DNA sequences which encode a library of millions of random peptides. Initially an excess number of virus compared to eukaryotic cells can be used. This leads to expression of a number of different peptides within each eukaryotic cell. These cells can subsequently be screened as described below, and the DNA can be isolated e.g. by PCR and used for reinfection of other cells. If an appropriate ratio between the number of retrovirus containing the random DNA sequences and cells is chosen, each cell will be transduced with a different random DNA sequence ("one cell - one ribonucleic acid or peptide"). This eventually enables the identification of an active peptide.

The peptide may optionally be targeted to different compartments in the cell by incorporating appropriate signal sequences in the translated sequences.

### EXAMPLE 2

### Identification of T cell epitopes by the use of mammalian intracellular expression libraries

The interleukin 2 (Il-2) dependent cell line, CTLL-2, is transfected with the murine Major Histocompatibility (MHC) class I molecule, K^{b}. Subsequently this cell line is infected with a retrovirus peptide library which was described in Example 1. This CTLL-2 peptide library expresses a wide range of random peptides, and if appropriate K^{b} associated anchor residues known to be important for peptide binding to K^{b} are introduced in the retroviral peptide sequence, a large library of peptides bound to K^{b} are presented on the surface of the CTLL-2 cells.

K^{b} restricted T cell hybridomas are generated against an appropriate virus antigen using conventional cellular immunological technology (Current Protocols in Immunology, Eds. Coligan et al., NIH). Such hybridomas secrete Il-2 upon recognition of antigen. A T cell hybridoma, which recognizes an unknown K^{b} bound virus T cell epitope, is subsequently incubated with samples of the K^{b} CTLL-2 library. If the hybridoma recognizes a peptide, the CTLL-2 cell presenting the peptide will be stimulated to proliferate by the Il-2 secreted by the hybridoma. In that way the CTLL-2 cell expressing the unknown virus T cell epitope can be selected and cloned. From this clone the DNA sequence encoding the peptide epitope in question can be isolated using PCR technology followed by conventional DNA sequencing. This eventually leads to identification of the unknown virus T cell epitope.

### EXAMPLE 3

### Identification of biologically active peptides or ribonucleic acids which regulate cell surface expression of proteins

Cells expressing the immunoregulatory membrane molecule, B7, are infected with the random peptide libraries constructed as described in Example 1. In this example a random eight-mer peptide library is introduced.

Using specific monoclonal antibodies the expression of B7 is analyzed by conventional methods. Cells which up- or down-regulate B7 can be selected either positively, e.g. using Fluorescence Activated Cell Sorting, or negatively, e.g. using appropriate antibodies in combination with lysis by complement.

Cells which show changes in expression of B7 are cloned by conventional means, and the DNA introduced by retroviral vectors is isolated using PCR and a set of retrovirus specific primers. The peptide sequence or possibly the RNA corresponding to said DNA may be able to modify the expression of B7 and hence the activation of T cells. This can subsequently be tested in conventional T cell assays.

### EXAMPLE 4

### Identification of a F(ab) fragment capable of modifying the immunoregulatory molecule B7.

A retroviral library encoding the variable heavy chain (V_{H}) as well as the variable light (V_{L}) gene fragments of the immunoglobulin molecule is produced. The gene region of both gene fragments corresponding to the antigen binding site of the resulting F(ab) fragments contains furthermore partly random gene sequences as described in example 1. This will lead to a large number of diverse peptide sequences in the antigen binding site of the F(ab) fragment. The retroviral vector library therefore encodes a large number of different F(ab) fragments with different antigen binding specification.

This library is subsequently transduced into a cell clone in such a way that each cell expresses a single F(ab) fragment species in e.g. the cytoplasm. Phenotypically changed cells are subsequently cloned and the sequence of the peptide in the intracellular (Fab) fragment is identified as described in example 1. This antibody can subsequently be produced in large scale by conventional means and be used for affinity purification of the cellular target protein responsible for the biological change of the cell phenotype. This can e.g. be done from lysates produced from the original non-modified cell clone.

Alternatively the retroviral F(ab) library can be constructed using e.g. a poly-His tag or other appropriate tags. In that way the antibody and the corresponding target can be isolated directly from the phenotypically changed cell by affinity chromatography. The isolated target can subsequently be identified by e.g. N-terminal amino acid sequencing in combination with conventional cloning methodology. Such target proteins are very important drug targets for further drug discovery.

## Claims

1. A method for identification of biologically active ribonucleic acids or peptides or for isolation of cellular ligands to the biologically active ribonucleic acids or peptides, which comprises the steps of
(a) production of a pool of appropriate vectors each containing a DNA sequence to be examined,
(b) efficient transduction of said vectors into a number of identical eukaryotic cells in such a way that each cell expresses either a single ribonucleic acid and possibly peptide encoded by the DNA sequence to be examined or a limited number of different ribonucleic acids and peptides encoded by DNA sequences to be examined,
(c) screening of said transduced cells to see whether some of them have changed a certain phenotypic trait, and
(d) selection and cloning of said changed cells,
characterized in that
the pool of appropriate vectors in step (a) contains totally or partly random DNA sequences selected from the group consisting of:
i) synthetic random DNA sequences obtainable by conventional random oligonucleotide synthesis;
ii) synthetic random DNA sequences wherein stop codons are absent and which encode random amino acid sequences with an even distribution of amino acids;
iii) synthetic DNA sequences defined in(i) or (ii) including codons which enable specific post-translational modifications of all expressed peptides or which encode anchor residues;
iv) synthetic DNA sequences defined in (i), (ii), or (iii) fused to or having inserted therein coding sequences for purification tags which facilitate the purification and identification of expressed peptides; and
v) synthetic DNA sequences defined in (i), (ii), (iii), or (iv) inserted into or fused to the coding sequence of a protein;
and wherein
(e) the vector DNA in the phenotypically changed cells is isolated and sequenced, and the sequences of the biologically active ribonucleic acids or peptides are deduced from the sequenced vector DNA;
and/or
(f) the biologically active ribonucleic acids or peptides expressed in the phenotypically changed cells are used directly for isolation of a ligand molecule to said ribonucleic acids or peptides.

2. A method according to claim 1, wherein the identical eukaryotic cells are cells of a cell line.

3. A method according to claim 1 or 2, in which the peptide is a peptide sequence introduced into or fused to a protein.

4. A method according to claim 3, wherein the protein is a F(ab) fragment or an antibody molecule.

5. A method according to any of claims 1-4, in which the synthetic DNA sequences/oligonucleotides have been produced by random codon synthesis, where defined DNA codons are synthesized in a random order.

6. A method according to claim 1-4, in which the synthetic DNA sequences/oligonucleotides have been produced by conventional random oligonucleotide synthesis.

7. A method according to any one of claims 1-6, in which the random DNA sequences are introduced into the expression vector by site directed PCR-mediated mutagenesis hereby ensuring the complexity of the totally or partly random DNA sequences.

8. A method according to claim 7 in which 3'-5' exonuclease trimming of PCR product 3' ends is used for optimal combining efficiencies of two PCR products.

9. A method according to any one of claims 1-8, in which the random DNA sequences are introduced into the number of eukaryotic cells in such a way that only one DNA sequence is introduced in each cell, one cell expressing one ribonucleic acid and possibly one peptide, thus enabling a particular sequence to by isolated and analysed.

10. A method according to any one of claims 1-9, in which the random DNA sequences are introduced into the eukaryotic cells by the use of appropriate viral vectors selected from retrovirus vectors and vaccinia virus vectors.

11. A method according to claim 10, in which the vector used is a retroviral vector.

12. A method according to claim 10, in which the vector used is a vaccinia virus vector.

13. A method according to claim 11, in which the retroviral vector contains a CMV promoter replacing the viral promoter in the 5'-LTR.

14. A method according to any one of claims 10-13, in which the random DNA sequences are produced as linear PCR products which are directly introduced into virus packaging cells by non-viral transfection methods.

15. A method according to any one of claims 10-14, in which the viral DNA introduced into the cells is amplified directly by PCR and used for retransfection of packaging cells to produce virus subsequently used for transduction of new cells with the purpose of eliminating false positives and/or enabling the "one cell - one ribonucleic acid or peptide" concept.

16. A method according to any one of claims 10-15, in which the viral titer of retroviral packaging cell lines is increased by transient transfection with a functional tRNA gene corresponding to the PBS in the vector.

17. A method according to any one of claims 10-16, in which is used a packaging cell line constructed from a vector expressing a single transcript which is translated into the three polyproteins/proteins, gag-pol, a drug resistance marker, and env.

18. A method according to any one of claims 10-17, in which is used a semi-packaging cell line with a corresponding minivirus-vector which enables vector expression after transduction rather than after transfection of cells.

19. A method according to any one of claims 1-18, wherein the expressed peptides are post-translationally modified or include anchor residues.

20. A method according to claim 19, wherein the post-translational modifications comprise introduction of glycosylation sites.

21. A method according to any one of claims 1-20, in which the biologically active peptide or expressed protein containing the random peptide also contains a purification tag enabling the direct isolation of the biologically active protein as well as the target protein causing the biological activity.

22. A method according to any one of claims 1-21, in which appropriate signal peptides, other leader molecules or recognition sequences also are encoded by the introduced DNA in such a way that they are fused to the expressed random peptides, or the expressed proteins containing the random peptide sequences, enabling these to be directed towards defined cellular compartments.

23. A method according to any one of claims 1-22, in which the random DNA sequences are introduced into, or fused to a DNA sequence encoding a protein expressed simultaneously with the random DNA sequences from the library vectors.

24. A method according to claim 23, in which the protein is selected from the group consisting of secreted proteins, intracellular proteins, and membrane proteins e.g. signal transducing molecules.

25. A method according to claim 23 or 24, in which the protein is derived wholly or partly from the heavy and/or light chain of an antibody molecule.

26. A method according to any one of claims 1-25, which is used for identification of T cell epitopes.

27. A method according to any one of claims 1-25, which is used for identifying biologically active peptides which regulate expression of cell surface proteins.

28. A method according to any of the preceding claims, wherein the identical eukaryotic cells are mammalian cells.

29. A method according to any of the preceding claims, wherein the random DNA sequences encode 6-10 random amino acids.

30. A drug development method which comprises the identification of biologically active ribonucleic acids or peptides or isolation of cellular ligands thereto according to the method of any of claims 1-28.

31. The method according to claim 30, wherein the identified biologically active ribonucleic acids or peptides serve as lead compounds.

## Patentansprüche

1. Verfahren zur Identifizierung von biologisch aktiven Ribonukleinsäuren oder Peptiden oder zur Isolierung zellulärer Liganden für die biologisch aktiven Ribonukleinsäuren oder Peptide, umfassend die Stufen
(a) Herstellung eines Pools geeigneter Vektoren, von denen jeder eine zu untersuchende DNA-Sequenz enthält,
(b) effiziente Transduktion der Vektoren in eine Anzahl identischer eukaryontischer Zellen in der Weise, daß jede Zelle entweder eine einzelne Ribonukleinsäure und gegebenenfalls ein durch die zu untersuchende DNA-Sequenz codiertes Peptid oder eine begrenzte Anzahl verschiedener Ribonukleinsäuren und durch die zu untersuchende DNA-Sequenzen codierte Peptide exprimiert,
(c) Screenen der transduzierten Zellen, um festzustellen, ob einige von ihnen ein bestimmtes phänotypisches Merkmal verändert haben und
(d) Selektieren und Clonieren der veränderten Zellen,
dadurch **gekennzeichnet,** daß
der Pool geeigneter Vektoren in der Stufe (a) vollständig oder teilweise Zufalls-DNA-Sequenzen enthält, die ausgewählt sind aus der Gruppe bestehend aus:
i) synthetischen Zufalls-DNA-Sequenzen, erhältlich durch konventionelle Zufallsoligonukleotidsynthese;
ii) synthetischen Zufalls-DNA-Sequenzen, in denen Stop-Codons fehlen und die Zufallsaminosäuresequenzen mit gleichmäßiger Aminosäureverteilung codieren;
iii) synthetischen DNA-Sequenzen wie in i) oder ii) definiert, enthaltend Codons, die spezifische posttranslationelle Modifikationen aller exprimierten Peptide ermöglichen oder die Ankerreste codieren;
iv) synthetischen DNA-Sequenzen wie in i), ii) oder iii) definiert, die mit bzw. in die Codesequenzen für Reinigungsmarkierungen, die die Reinigung und Identifizierung der exprimierten Peptide erleichtern, fusioniert oder insertiert sind; und
v) synthetischen DNA-Sequenzen wie in i), ii), iii) oder iv) definiert, die in bzw. mit eine(r) codierende(n) Sequenz für ein Protein insertiert oder fusioniert sind;
und worin
(e) die Vektor-DNA in den phänotypisch veränderten Zellen isoliert und sequenziert wird und die Sequenzen der biologisch aktiven Ribonukleinsäuren oder Peptide aus der sequenzierten Vektor-DNA abgeleitet werden;
und/oder
(f) die biologisch aktiven Ribonukleinsäuren oder in den phänotypisch veränderten Zellen exprimierten Peptide direkt zur Isolierung eines Ligandenmoleküls für die Ribonukleinsäuren oder Peptide verwendet werden.

2. Verfahren nach Anspruch 1, dadurch **gekennzeichnet,** daß die identischen eukaryontischen Zellen Zellen einer Zellinie sind.

3. Verfahren nach Anspruch 1 oder 2, dadurch **gekennzeichnet,** daß das Peptid eine in ein Protein eingeführte oder damit fusionierte Peptidsequenz ist.

4. Verfahren nach Anspruch 3, dadurch **gekennzeichnet,** daß das Protein ein F(ab)-Fragment oder ein Antikörpermolekül ist.

5. Verfahren nach einem der Ansprüche 1 bis 4, dadurch **gekennzeichnet,** daß die synthetischen DNA-Sequenzen/Oligonukleotide durch Zufallscodonsynthese hergestellt worden sind, wobei definierte DNA-Codons in zufälliger Reihenfolge synthetisiert werden.

6. Verfahren nach einem der Ansprüche 1 bis 4, dadurch **gekennzeichnet,** daß die synthetischen DNA-Sequenzen/Oligonukleotide durch konventionelle Zufallsoligonucleotidsynthese hergestellt worden sind.

7. Verfahren nach einem der Ansprüche 1 bis 6, dadurch **gekennzeichnet,** daß die Zufalls-DNA-Sequenzen in den Expressionsvektor durch ortsspezifische PCR-vermittelte Mutagenese eingeführt werden, wodurch die Komplexität der vollständigen oder teilweisen Zufalls-DNA-Sequenzen sichergestellt wird.

8. Verfahren nach Anspruch 7, dadurch **gekennzeichnet,** daß 3'-5'-Exonuclease-Trimming der 3'-Enden des PCR-Produkts verwendet wird zur Erzielung optimaler Kombinationseffizienzen von zwei PCR-Produkten.

9. Verfahren nach einem der Ansprüche 1 bis 8, dadurch **gekennzeichnet,** daß die Zufalls-DNA-Sequenzen in solcher Weise in eine Anzahl eukaryontischer Zellen eingeführt werden, daß in jede Zelle nur eine DNA-Sequenz eingeführt wird, wobei eine Zelle eine Ribonukleinsäure und gegebenenfalls ein Peptid exprimiert, wodurch die Isolierung und Analyse einer bestimmten Sequenz ermöglicht wird.

10. Verfahen nach einem der Ansprüche 1 bis 9, dadurch **gekennzeichnet,** daß die Zufalls-DNA-Sequenzen in die eukaryontischen Zellen eingeführt werden durch Verwendung geeigneter viraler Vektoren, ausgewählt aus Retrovirus-Vektoren und Vacciniavirus-Vektoren.

11. Verfahren nach Anspruch 10, dadurch **gekennzeichnet,** daß der verwendete Vektor ein retroviraler Vektor ist.

12. Verfahren nach Anspruch 10, dadurch **gekennzeichnet,** daß der verwendete Vektor ein Vacciniavirus-Vektor ist.

13. Verfahren nach Anspruch 11, dadurch **gekennzeichnet,** daß der retrovirale Vektor einen CMV-Promotor enthält, der den viralen Promotor in dem 5'-LTR ersetzt.

14. Verfahren nach einem der Ansprüche 10 bis 13, dadurch **gekennzeichnet,** daß die Zufalls-DNA-Sequenzen als lineare PCR-Produkte hergestellt werden, die durch nichtvirale Transfektionsmethoden direkt in die Virusverpackungszellen eingeführt werden.

15. Verfahren nach einem der Ansprüche 10 bis 14, dadurch **gekennzeichnet,** daß die in die Zellen eingeführte virale DNA direkt durch PCR amplifiziert und zur Retransfektion von Verpackungszellen verwendet wird, um Viren herzustellen, die anschließend zur Transduktion neuer Zellen verwendet werden zum Zweck der Eliminierung falscher Positiv-Ergebnisse und/oder zur Verwirklichung des "eine Zelle-eine Ribonukleinsäure oder Peptid"-Konzepts.

16. Verfahren nach einem der Ansprüche 10 bis 15, dadurch **gekennzeichnet,** daß der virale Titer der retroviralen Verpackungszellinien erhöht wird durch transiente Transfektion mit einem funktionellen tRNA-Gen, das der PBS in dem Vektor entspricht.

17. Verfahren nach einem der Ansprüche 10 bis 16, dadurch **gekennzeichnet,** daß eine Verpackungszellinie verwendet wird, die konstruiert ist aus einem Vektor, der ein einzelnes Transkript exprimiert, das in die drei Polyproteine/Proteine, gag-pol, einen Arzneimittelresistenzmarker und env translatiert wird.

18. Verfahren nach einem der Ansprüche 10 bis 17, dadurch **gekennzeichnet,** daß eine Halbverpackungszellinie verwendet wird mit einem entsprechenden Minivirusvektor, der die Vektorexpression eher nach der Transduktion als nach der Transfektion der Zellen ermöglicht.

19. Verfahren nach einem der Ansprüche 1 bis 18, dadurch **gekennzeichnet,** daß die exprimierten Peptide posttranslationell modifiziert werden oder Ankerreste einschließen.

20. Verfahren nach Anspruch 19, dadurch gekennzeichnet , daß die posttranslationellen Modifizierungen die Einführung von Glykosylierungsstellen umfaßt.

21. Verfahren nach einem der Ansprüche 1 bis 20, dadurch **gekennzeichnet,** daß das biologisch aktive Peptid oder exprimierte Protein, das das Zufallspeptid enthält, außerdem eine Reinigungsmarkierung enthält, die die direkte Isolierung des biologisch aktiven Proteins sowie des die biologische Aktivität verursachenden Zielproteins ermöglicht.

22. Verfahren nach einem der Ansprüche 1 bis 21, dadurch **gekennzeichnet,** daß geeignete Signalpeptide, andere Leadermoleküle oder Erkennungssequenzen ebenfalls durch die eingeführte DNA codiert werden in der Weise, daß sie mit den exprimierten Zufallspeptiden oder den exprimierten Proteinen, die die Zufallspeptidsequenzen enthalten, fusioniert sind, so daß diese auf definierte zelluläre Bereiche gerichtet werden können.

23. Verfahren nach einem der Ansprüche 1 bis 22, dadurch **gekennzeichnet,** daß die Zufalls-DNA-Sequenzen in bzw. mit DNA-Sequenzen, die ein gleichzeitig mit den Zufalls-DNA-Sequenzen aus den Bankvektoren exprimiertes Protein codieren, eingeführt oder fusioniert sind.

24. Verfahren nach Anspruch 23, dadurch gekennzeichnet , daß das Protein ausgewählt ist aus der Gruppe, bestehend aus sezernierten Proteinen, intrazellulären Proteinen und Membranproteinen, z.B. Signaltransduktions-Molekülen.

25. Verfahren nach Anspruch 23 oder 24, dadurch gekennzeichnet , daß das Protein ganz oder teilweise abgeleitet ist von der schweren und/oder leichten Kette eines Antikörpermoleküls.

26. Verfahren nach einem der Ansprüche 1 bis 25, dadurch **gekennzeichnet,** daß es zur Identifizierung von T-Zell-Epitopen verwendet wird.

27. Verfahren nach einem der Ansprüche 1 bis 25, dadurch **gekennzeichnet,** daß es für die Identifizierung biologisch aktiver Peptide, die die Expression von Zelloberflächenproteinen regulieren, verwendet wird.

28. Verfahren nach einem der vorstehenden Ansprüche, dadurch **gekennzeichnet,** daß die identischen eukaryontischen Zellen Säugetierzellen sind.

29. Verfahren nach einem der vorstehenden Ansprüche, dadurch **gekennzeichnet,** daß die Zufalls-DNA-Sequenzen 6 bis 10 Zufallsaminosäuren codieren.

30. Wirkstoff-Entwicklungsverfahren, umfassend die Identifizierung biologisch aktiver Ribonukleinsäuren oder Peptide oder die Isolierung zellulärer Liganden dafür gemäß dem Verfahren nach einem der Ansprüche 1 bis 28.

31. Verfahren nach Anspruch 30, dadurch **gekennzeichnet,** daß die identifizierten biologisch aktiven Ribonukleinsäuren oder Peptide als Leaderverbindungen dienen.

## Revendications

1. Procédé d'identification d'acides ribonucléiques ou de peptides biologiquement actifs, ou pour l'isolement de ligands cellulaires pour les acides ribonucléiques ou peptides biologiquement actifs, qui comprend les étapes de
(a) production d'un groupe de vecteurs appropriés contenant chacun une séquence d'ADN à examiner,
(b) transduction efficace desdits vecteurs dans un certain nombre de cellules eucaryotes identiques de telle manière que chaque cellule exprime soit un seul acide ribonucléique et éventuellement peptide codé par la séquence d'ADN à examiner, soit un nombre limité de différents acides ribonucléiques et peptides codés par les séquences d'ADN à examiner,
(c) criblage desdites cellules transduites pour voir si certaines de celles-ci présentent un certain caractère phénotypique modifié, et
(d) sélection et clonage desdites cellules modifiées,
caractérisé en ce que
le groupe de vecteurs appropriés dans l'étape (a) contient des séquences d'ADN partiellement ou totalement aléatoires choisies dans l'ensemble constitué par:
I) des séquences d'ADN synthétiques aléatoires pouvant être obtenues par classique synthèse aléatoire d'oligonucléotides;
II) des séquences d'ADN synthétiques aléatoires dans lesquelles des codons d'arrêt sont absents et qui codent des séquences aléatoires d'aminoacides avec une répartition régulière d'aminoacides;
III) des séquences d'ADN synthétiques définies en (I) ou (II) comprenant des codons qui permettent certaines modifications post-traductionnelles de tous les peptides exprimés ou qui codent des résidus d'ancrage;
IV) des séquences d'ADN synthétiques définies en (I), (II) ou (III) fusionnées avec ou comportant insérées des séquences codant pour des étiquettes de purification qui facilitent la purification et l'identification de peptides exprimés; et
V) des séquences d'ADN synthétiques définies en (I), (II), (III) ou (IV), insérées dans ou fusionnées avec la séquence codante d'une protéine;
et dans lequel
(e) l'ADN vecteur dans les cellules à phénotype modifié est isolé et séquencé, et les séquences des acides ribonucléiques ou peptides biologiquement actifs sont déduites de l'ADN vecteur séquencé;
et/ou
(f) les acides ribonucléiques ou peptides biologiquement actifs exprimés dans les cellules à phénotype modifié sont utilisés directement pour l'isolement d'une molécule de ligand pour lesdits acides ribonucléiques ou peptides.

2. Procédé selon la revendication 1, dans lequel les cellules eucaryotes identiques sont des cellules d'une lignée cellulaire.

3. Procédé selon la revendication 1 ou 2, dans lequel le peptide est une séquence peptidique introduite dans ou fusionnée avec une protéine.

4. Procédé selon la revendication 3, dans lequel la protéine est un fragment F(ab) ou une molécule d'anticorps.

5. Procédé selon l'une quelconque des revendications 1 à 4, dans lequel les oligonucléotides/séquences d'ADN synthétiques ont été produits par synthèse aléatoire de codons, où des codons d'ADN définis sont synthétisés dans un ordre aléatoire.

6. Procédé selon l'une quelconque des revendications 1 à 4, dans lequel les oligonucléotides/séquences d'ADN synthétiques ont été produits par synthèse aléatoire d'oligonucléotides classique.

7. Procédé selon l'une quelconque des revendications 1 à 6, dans lequel les séquences d'ADN aléatoires sont introduites dans le vecteur d'expression par mutagénèse dirigée sur un site, médiée par PCR (réaction d'amplification en chaîne par polymérase), ce qui assure la complexité des séquences d'ADN partiellement ou totalement aléatoires.

8. Procédé selon la revendication 7, dans lequel un rognage par une 3'-5'-exonucléase des extrémités 3' du produit de PCR est utilisé pour des rendements optimaux de combinaison de deux produits de PCR.

9. Procédé selon l'une quelconque des revendications 1 à 8, dans lequel les séquences d'ADN aléatoires sont introduites dans le nombre de cellules eucaryotes de telle façon qu'une seule séquence d'ADN est introduite dans chaque cellule, une cellule exprimant un acide ribonucléique et éventuellement un peptide, ce qui permet l'isolement et l'analyse d'une séquence particulière.

10. Procédé selon l'une quelconque des revendications 1 à 9, dans lequel les séquences d'ADN aléatoires sont introduites dans les cellules eucaryotes par l'utilisation de vecteurs viraux appropriés choisis parmi des vecteurs rétroviraux et des vecteurs de type virus de la vaccine.

11. Procédé selon la revendication 10, dans lequel le vecteur utilisé est un vecteur rétroviral.

12. Procédé selon la revendication 10, dans lequel le vecteur utilisé est un vecteur de type virus de la vaccine.

13. Procédé selon la revendication 11, dans lequel le vecteur rétroviral contient un promoteur de CMV (cytomégalovirus) remplaçant le promoteur viral dans la 5'-LTR (longue répétition terminale).

14. Procédé selon l'une quelconque des revendications 10 à 13, dans lequel les séquences d'ADN aléatoires sont produites sous forme de produits linéaires de PCR qui sont directement introduits dans des cellules encapsidant des virus, par des méthodes de transfection non virale.

15. Procédé selon l'une quelconque des revendications 10 à 14, dans lequel l'ADN viral introduit dans les cellules est amplifié directement par PCR et utilisé pour la retransfection de cellules encapsidantes pour produire des virus ultérieurement utilisés pour la transduction de nouvelles cellules dans le but d'éliminer des faux positifs et/ou de permettre le concept "une cellule - un acide ribonucléique ou peptide".

16. Procédé selon l'une quelconque des revendications 10 à 15, dans lequel le titre viral de lignées cellulaires encapsidant des rétrovirus est accru par transfection transitoire avec un gène d'ARNt fonctionnel correspondant au segment PBS dans le vecteur.

17. Procédé selon l'une quelconque des revendications 10 à 16, dans lequel on utilise une lignée de cellules encapsidantes construite à partir d'un vecteur exprimant un seul transcrit qui est traduit en les trois polypeptides/protéines, gag-pol, un marqueur de résistance à des médicaments, et env.

18. Procédé selon l'une quelconque des revendications 10 à 17, dans lequel est utilisée une lignée cellulaire semi-encapsidante avec un vecteur-minivirus correspondant qui permet l'expression du vecteur après transduction, plutôt qu'après transfection des cellules.

19. Procédé selon l'une quelconque des revendications 1 à 18, dans lequel les peptides exprimés sont modifiés par modification post-traductionnelle ou comprennent des résidus d'ancrage.

20. Procédé selon la revendication 19, dans lequel les modifications post-traductionnelles comprennent l'introduction de sites de glycosylation.

21. Procédé selon l'une quelconque des revendications 1 à 20, dans lequel le peptide biologiquement actif ou la protéine exprimée contenant le peptide aléatoire contient également une étiquette de purification permettant l'isolement direct de la protéine biologiquement active ainsi que de la protéine cible provoquant l'activité biologique.

22. Procédé selon l'une quelconque des revendications 1 à 21, dans lequel des peptides signaux appropriés, d'autres molécules de tête ou des séquences de reconnaissance sont également codés par l'ADN introduit de telle sorte qu'ils sont fusionnés avec les peptides aléatoires exprimés, ou les protéines exprimées contenant les séquences peptidiques aléatoires, ce qui permet de les diriger vers des compartiments cellulaires définis.

23. Procédé selon l'une quelconque des revendications 1 à 22, dans lequel les séquences d'ADN aléatoires sont introduites dans ou fusionnées avec une séquence d'ADN codant pour une protéine exprimée en même temps que les séquences d'ADN aléatoires provenant des vecteurs de la banque.

24. Procédé selon la revendication 23, dans lequel la protéine est choisie dans l'ensemble constitué par des protéines sécrétées, des protéines intracellulaires et des protéines membranaires, par exemple, des molécules transductrices de signal.

25. Procédé selon la revendication 23 ou 24, dans lequel la protéine est issue totalement ou en partie de la chaîne lourde et/ou de la chaîne légère d'une molécule d'anticorps.

26. Procédé selon l'une quelconque des revendications 1 à 25, qui est utilisé pour l'identification d'épitopes de lymphocytes T.

27. Procédé selon l'une quelconque des revendications 1 à 25, qui est utilisé pour identifier des peptides actifs qui régulent l'expression de protéines de surface cellulaire.

28. Procédé selon l'une quelconque des revendications précédentes, dans lequel les cellules eucaryotes identiques sont des cellules mammaliennes.

29. Procédé selon l'une quelconque des revendications précédentes, dans lequel les séquences d'ADN aléatoires codent pour 6-10 aminoacides.

30. Procédé de développement d'un médicament, qui comprend l'identification d'acides ribonucléiques ou de peptides biologiquement actifs ou l'isolement de ligands cellulaires pour ceux-ci, selon le procédé de l'une quelconque des revendications 1 à 28.

31. Procédé selon la revendication 30, dans lequel les acides ribonucléiques ou peptides biologiquement actifs identifiés servent de composés directeurs.
